# EUROPÄISCHE PATENTANMELDUNG

(11) **EP 4 427 764 A1**
(43) Veröffentlichungstag der Anmeldung: **11.09.2024**
(21) Anmeldenummer: 23160940.5
(22) Anmeldetag: 09.03.2023
(51) Int. Cl.: A61L 2/04, A61C 1/00

(54) **LASERSYSTEM ZUM ENTFERNEN VON BAKTERIEN**

(71) Anmelder: Ivoclar Vivadent AG, 9494 Schaan (LI)
(72) Erfinder: Grosse-Honebrink, Alexander, 9320 Arbon (CH); Reinhardt, Jonas, 7206 Igis (CH); Tichy, Raimund, 6800 Feldkirch (AT); Van den Heuvel, Erik, 1402 CG Bussum (NL); De Josselin de Jong, Elbert, 1402 CG Bussum (NL)
(74) Vertreter: Baldus, Oliver

(57) **Zusammenfassung**

Die vorliegende Erfindung betrifft ein Lasersystem (100) zum Entfernen von Bakterien, mit einer porösen Oxidkeramik (101); und einem Laser (103) zum Aussenden von Laserlichtpulsen auf die Oxidkeramik (101) mit einer Energie pro Fläche zwischen 1,8×10⁻¹¹ J/cm² und 1,5 J/cm².

## Beschreibung

Die vorliegende Erfindung betrifft ein Lasersystem zum Entfernen von Bakterien und ein Laserverfahren zum Entfernen von Bakterien von einer porösen Oxidkeramik.

Es ist die technische Aufgabe der Erfindung, Bakterien auf einer Oxidkeramik effizient zu entfernen.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Lasersystem zum Entfernen von Bakterien gelöst, mit einer porösen Oxidkeramik; und einem Laser zum Aussenden von Laserlichtpulsen auf die Oxidkeramik mit einer Energie pro Fläche zwischen 1,8×10⁻¹⁰ J/cm² und 1,5 J/cm². Durch den Laserlichtpuls mit der angegebenen Energie können Bakterien auf der Oxidkeramik deaktiviert und zerstört werden.

In einer technischen vorteilhaften Ausführungsform des Lasersystems ist der Laser ausgebildet, die Laserlichtpulse jeweils mit einer Dauer von unter 400 fs auszusenden. Dadurch wird beispielsweise der technische Vorteil erreicht, dass die Energie mit einer hohen Leistung auf die Oxidkeramik aufgebracht werden kann und Bakterien wirkungsvoll zerstört werden.

In einer weiteren technischen vorteilhaften Ausführungsform des Lasersystems ist der Laser ausgebildet, die Laserlichtpulse mit einer Wiederholrate von mehr als 10 MHz oder 80 MHz auszusenden. Dadurch wird beispielsweise der technische Vorteil erreicht, dass die Bakterien auf schnelle Weise und über eine große Fläche zerstört werden können.

In einer weiteren technischen vorteilhaften Ausführungsform des Lasersystems ist der Laser ausgebildet, die Laserlichtpulse jeweils mit einer Flächenleistungsdichte von über 1 kW/cm² auf die Oxidkeramik auszusenden. Dadurch wird beispielsweise ebenfalls der technische Vorteil erreicht, dass die Bakterien schnell bei geringer Wärmeentwicklung zerstört werden.

In einer weiteren technischen vorteilhaften Ausführungsform des Lasersystems ist der Laser ein Ti:Sa-Laser. Dadurch wird beispielsweise der technische Vorteil erreicht, dass sich die Laserpulse mit geringem Aufwand erzeugen lassen.

In einer weiteren technischen vorteilhaften Ausführungsform des Lasersystems weist die Oxidkeramik offene Poren mit einer Porengröße zwischen 0,5 pm bis 20 pm auf. Dadurch wird beispielsweise der technische Vorteil erreicht, dass die Zerstörung von Bakterien durch Zufuhr weiterer Substanzen weiter verbessert werden kann.

In einer weiteren technischen vorteilhaften Ausführungsform des Lasersystems weist die Oxidkeramik Kanäle auf, die sich von einer Oberfläche in das Innere der Oxidkeramik erstrecken. Dadurch wird beispielsweise der technische Vorteil erreicht, dass sich die Substanzen auf einfache Weise zuführen lassen.

In einer weiteren technischen vorteilhaften Ausführungsform des Lasersystems umfasst das Lasersystem eine Fluoreszenzanregungsvorrichtung zum Erzeugen von sichtbarem Fluoreszenzlicht durch die Bakterien auf der Oxidkeramik. Dadurch wird beispielsweise der technische Vorteil erreicht, dass eine Kontamination durch Bakterien erkannt werden kann.

In einer weiteren technischen vorteilhaften Ausführungsform des Lasersystems umfasst das Lasersystem eine elektronische Kamera zum Erfassen des Fluoreszenzlichts. Dadurch wird beispielsweise der technische Vorteil erreicht, dass elektrische Signale zum Erfassen der Kontamination erzeugt werden können.

In einer weiteren technischen vorteilhaften Ausführungsform des Lasersystems ist die elektronische Kamera ausgebildet, diejenigen Bereiche der Oxidkeramik zu erfassen, von denen das Fluoreszenzlicht ausgesendet wird. Dadurch wird beispielsweise der technische Vorteil erreicht, dass kontaminierte Bereiche erfasst und digital verarbeitet werden können.

In einer weiteren technischen vorteilhaften Ausführungsform des Lasersystems umfasst das Lasersystem eine Richtvorrichtung zum automatischen Richten der Laserpulse auf die erfassten Bereiche oder eine Aktivierungsvorrichtung zum Aktivieren des Lasers, wenn eine Fluoreszenz erfasst wird. Dadurch wird beispielsweise der technische Vorteil erreicht, dass die Laserpulse nicht unnötig auf unkontaminierte Bereiche treffen und die Effizienz des Lasersystems gesteigert wird.

In einer weiteren technischen vorteilhaften Ausführungsform des Lasersystems ist das Lasersystem ausgebildet, die Energie pro Fläche der Laserpulse auf Basis einer Intensität des erfassten Fluoreszenzlichtes zu steuern. Dadurch wird beispielsweise der technische Vorteil erreicht, dass Bereiche mit hoher Kontamination einer höheren Strahlung ausgesetzt werden und eine Zerstörung der Bakterien sichergestellt werden kann.

In einer weiteren technischen vorteilhaften Ausführungsform des Lasersystems umfasst das Lasersystem einen Polarisator zum Polarisieren des Laserlichts. Dadurch wird beispielsweise der technische Vorteil erreicht, dass polarisiertes Licht erzeugt werden kann, das tief in die Oxidkeramik eindringt und nur wenig von der Oberfläche reflektiert wird.

In einer weiteren technischen vorteilhaften Ausführungsform des Lasersystems ist der Polarisator drehbar. Dadurch wird beispielsweise der technische Vorteil erreicht, dass die Polarisation je nach Winkel der Oxidkeramik zum Laserstrahl eingestellt werden kann.

Gemäß einem zweiten Aspekt wird die technische Aufgabe durch ein Laserverfahren zum Entfernen von Bakterien von einer porösen Oxidkeramik gelöst, mit dem Schritt eines Aussendens von Laserlichtpulsen auf die Oxidkeramik mit einer Energie pro Fläche zwischen 1,8×10⁻¹⁰ J/cm² und 1,5 J/cm². Durch das Verfahren werden die gleichen technischen Vorteile wie durch das Lasersystem nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Darstellung eines Lasersystems zum Entfernen von Bakterien; und
- Fig. 2: ein Blockdiagramm eines Verfahrens zum Entfernen von Bakterien von einer porösen Oxidkeramik.

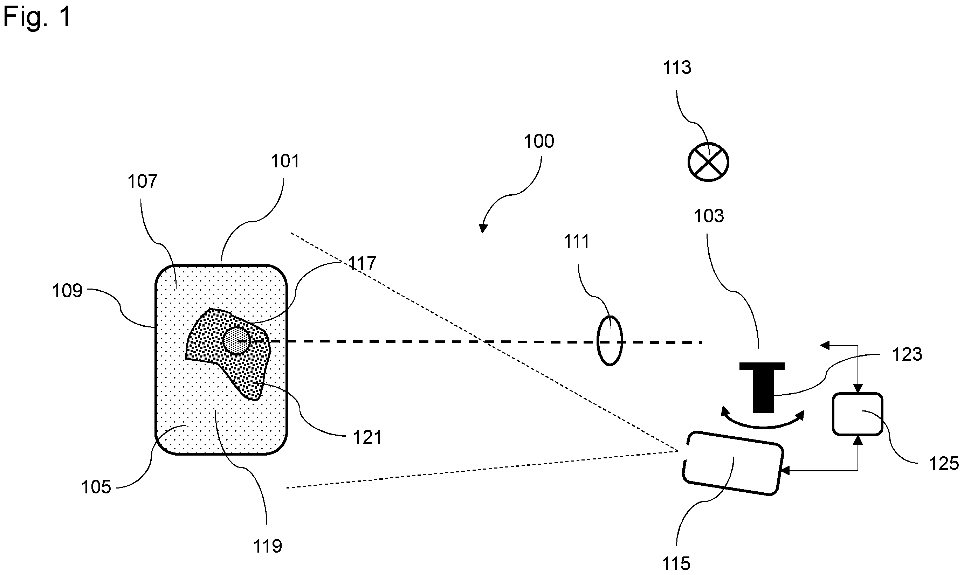

Fig. 1 zeigt eine schematische Darstellung eines Lasersystems 100 zum Entfernen von Bakterien 121 von einer porösen Oxidkeramik 101. Die Oxidkeramik 101 umfasst offene Poren 119 mit einer Porengröße zwischen 0,5 pm bis 20 um. Die Poren 119 können durch Kanäle 105 gebildet sein, die sich von einer Oberfläche 107 in das Innere 109 der Oxidkeramik 101 erstrecken. Die Bakterien 121 haften auf einer Oberfläche der Oxidkeramik 101, in einem kontaminierten Bereich 117 an.

Das Lasersystem 100 umfasst eine poröse Oxidkeramik 101 und einen Laser 103 zum Aussenden von Laserlichtpulsen auf die Oxidkeramik 101 mit je einer Energie pro Fläche zwischen 1,8×10⁻¹⁰ J/cm² und 1,5 J/cm². Die Oxidkeramik 101 und der Laser 103 sind derart angeordnet und abgestimmt, dass jeder Laserpuls, der auf die Oberfläche 107 der Oxidkeramik 101 auftrifft, dort eine Energie pro Fläche zwischen 1,8×10⁻¹⁰ J/cm² und 1,5 J/cm² aufweist. In diesem Bereich lassen sich die Bakterien 121 besonders wirksam durch den Laser deaktivieren.

Der Laser 103 ist beispielsweise ein Ti:Sa-Laser, der Laserlichtpulse mit einer Dauer von unter 400 fs aussendet, beispielsweise zwischen 100 und 200 fs (Femtosekundenlaser). Der Ti:Sa-Laser ist ein Festkörperlaser, der als optisch aktives Medium die Fluoreszenz von Titan-Ionen benutzt, die als Dotierung in einem Korund (Al2O3)-Kristall vorliegen. Die Wellenlänge des Lasers 103 ist durchstimmbar und liegt beispielsweise zwischen 670-1070 nm. Besonders vorteilhaft ist, wenn die Wellenlänge 370-420 nm beträgt. In diesem Bereich dringt das Laserlicht nur wenig in die Oxidkeramik ein, so dass Bakterien an der Oberfläche wirksam zerstört werden.

Die Laserlichtpulse des Lasers 103 werden mit einer Wiederholrate von mehr als 1×10⁷ oder 8×10⁷ Lichtpulsen/Sekunde ausgesendet. Der Laser 103 sendet die Laserlichtpulse beispielsweise jeweils mit einer Flächenleistungsdichte von über 1kW/cm² auf die Oxidkeramik 101 aus.

Um die Bakterien 121 auf der Oberfläche 107 der Oxidkeramik 101 sichtbar zu machen, umfasst das Lasersystem 100 eine Fluoreszenzanregungsvorrichtung 113 zum Erzeugen von sichtbarem Fluoreszenzlicht. Die Fluoreszenzanregungsvorrichtung 113 ist beispielsweise durch einen weiteren Laser oder eine Leuchtdiode gebildet. Die Fluoreszenzanregungsvorrichtung 113 emittiert beispielsweise kontinuierliche Strahlung mit einer Wellenlänge zwischen 380 und 415 nm, besonders bevorzugt 405 nm. Die Fluoreszenzanregungsvorrichtung 113 kann gesteuert ein- und ausgeschaltet werden.

Diese Strahlung der Fluoreszenzanregungsvorrichtung 113 trifft auf die Oxidkeramik 101 und regt dort die Bakterien 121 in dem kontaminierten Bereich 117 zur Fluoreszenz an. Fluoreszenz ist die spontane Emission von Licht kurz nach der Anregung eines Bakteriums 121 durch Licht. Dabei sind die emittierten Photonen in der Regel energieärmer als die vorher absorbierten. Durch das charakteristische Fluoreszenzlicht können kontaminierte Bereiche 121 der Oxidkeramik 101 erkannt werden, da nicht kontaminierte Bereiche kein Fluoreszenzlicht aussenden. Aufgrund der verschobenen Wellenlänge des Fluoreszenzlichtes können Bereiche 117 mit Bakterien gut erkannt werden.

Zum Erfassen des Fluoreszenzlichtes umfasst das Lasersystems 100 eine elektronische Kamera 115, mit der diejenigen Bereiche 117 der Oxidkeramik 101 erfasst werden können, von denen das Fluoreszenzlicht ausgesendet wird. Die Kamera 115 ist dazu auf die Oxidkeramik 101 gerichtet. Die elektronische Kamera 115 umfasst beispielsweise ein CCD-Feld, um ein digitales Bild von der Oberfläche der Oxidkeramik 101 aufzunehmen. Vor der Kamera 115 kann ein geeignetes Hochpassfilter angeordnet sein, beispielsweise mit einer Grenzwellenlänge von 450 oder 550 nm.

In diesem digitalen Bild können die kontaminierten Bereiche 117 der Oxidkeramik 101 erfasst werden. Das digitale Bild umfasst dabei nicht nur Positionsinformationen, sondern auch Helligkeitsinformationen (Intensitätsinformationen) des von den Bakterien 121 ausgesendeten Fluoreszenzlichtes. Das digitale Bild kann durch einen Microprozessor weiterverarbeitet und ausgewertet werden. Dadurch lassen sich beispielsweise die Koordinaten derjenigen Bereiche auf der Oberfläche 107 der Oxidkeramik 101 ermitteln, von denen das Fluoreszenzlicht ausgesendet wird. Zudem lässt sich erfassen, in welcher Intensität das Fluoreszenzlicht von den Positionen auf der Oberfläche der Oxidkeramik 101 ausgesendet wird. Die Intensität des Fluoreszenzlichtes entspricht dabei dem Befall mit Bakterien 121.

Das Lasersystem 100 umfasst beispielsweise eine Richtvorrichtung 123 zum automatischen Richten der Laserpulse auf die erfassten Bereiche 117. Die Richtvorrichtung 123 ist beispielsweise in der Lage, den Laser durch eine mechanische Bewegung oder Drehung auf die kontaminierten Bereiche 117 zu richten. Dazu wertet die Richtvorrichtung das digitale Bild von der Oberfläche der Oxidkeramik 101 aus und bestimmt die Position der kontaminierten Bereiche 117, beispielsweise in Form von X- und Y-Koordinaten. Auf diese Bereiche richtet oder lenkt die Richtvorrichtung 123 den Laserstrahl.

Auf diese Weise werden die Laserpulse selektiv auf die kontaminierten Bereiche gelenkt. Im Allgemeinen kann die Richtvorrichtung auch durch andere Maßnahmen realisiert sein, wie beispielsweise über steuerbare Umlenkspiegel.

Zudem kann auf Basis des erfassten digitalen Bildes von der Oberfläche 107 der Oxidkeramik 101 eine Intensität der ausgesendeten Laserlichtpulse gesteuert werden. Wird eine hohe Intensität des Fluoreszenzlichtes an einer Position erfasst, beispielsweise wenn die Intensität des Fluoreszenzlichtes über einem vorgegebenen Grenzwert liegt, wird beispielsweise die Energie pro Fläche und/oder die Wiederholrate der Laserlichtpulse erhöht.

Wird demgegenüber eine niedrige Intensität des Fluoreszenzlichtes an einer Position erfasst, beispielsweise wenn die Intensität des Fluoreszenzlichtes unter einem vorgegebenen Grenzwert liegt, wird beispielsweise die Energie pro Fläche und/oder die Wiederholrate verringert.

Insbesondere kann eine elektronische Aktivierungsvorrichtung 125 den Laser 103 nur dann aktivieren, wenn die Intensität des Fluoreszenzlichtes an einer Position, auf die der Laser 101 gerichtet ist, über einem vorgegebenen Schwellwert liegt. Liegt die Intensität des Fluoreszenzlichtes an der Position, auf die der Laser 103 gerichtet ist, unter dem vorgegebenen Schwellwert, wird der Laser deaktiviert. In diesem Fall kann der Laser 101 wahlweise auf unterschiedliche Bereiche oder Positionen der Oxidkeramik 101 gerichtet werden und schaltet sich nur dann ein, wenn sich dort Bakterien 121 befinden.

Zudem kann das Lasersystem 100 einen Polarisator 111 zum Polarisieren des Laserlichts umfassen. Durch den Polarisator 111 wird das Laserlicht in einer bestimmten Ebene polarisiert. Der Polarisator 111 kann drehbar gelagert sein und durch das Lasersystem 100 in seiner Stellung gesteuert werden. Je nach dem Winkel, in dem die Laserlichtpulse auf die Oxidkeramik 101 auftreffen, wird auf Basis der Bragg-Beziehung eine Polarisation gewählt, bei der der Laserlichtpuls möglichst wenig von der Oberfläche 107 reflektiert wird und/oder möglichst tief in die Oxidkeramik eindringt.

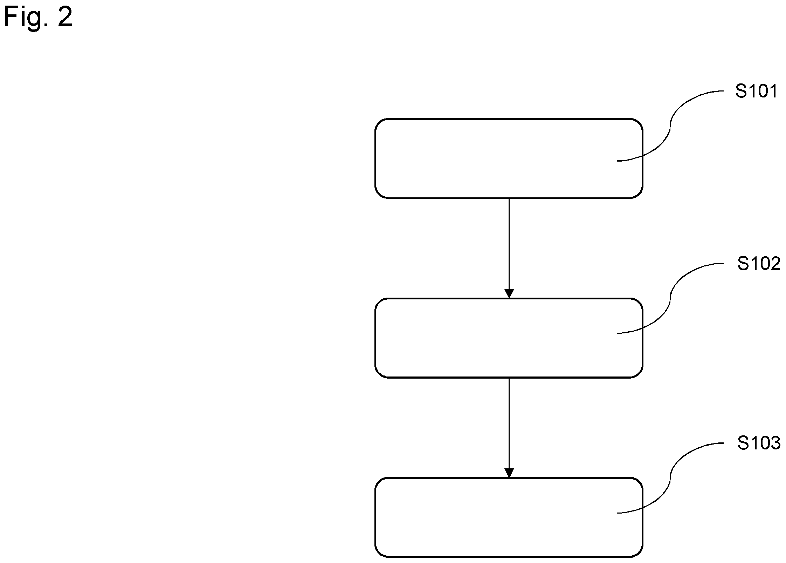

Fig. 2 zeigt ein Blockdiagramm eines Verfahrens zum Entfernen von Bakterien 121 von einer porösen Oxidkeramik 101. In Schritt S101 können zuerst diejenigen Bereiche 117 der Oxidkeramik 101 erfasst werden, von denen das Fluoreszenzlicht ausgesendet wird. Danach wird der Laser 103 durch die Richtvorrichtung 123 auf diese Bereiche 107 ausgerichtet. In Schritt S103 werden Laserlichtpulse auf die Oxidkeramik 101 mit einer Energie pro Fläche zwischen 1,8×10⁻¹⁰ J/cm² und 1,5 J/cm² ausgesendet, um die Bakterien abzutöten.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Lasersystem
- 101: Oxidkeramik
- 103: Laser
- 105: Kanal
- 107: Oberfläche
- 109: Innere
- 111: Polarisator
- 113: Fluoreszenzanregungsvorrichtung
- 115: elektronische Kamera
- 117: Bereiche
- 119: Poren
- 121: Bakterien
- 123: Richtvorrichtung
- 125: Aktivierungsvorrichtung

## Patentansprüche

1. Lasersystem (100) zum Entfernen von Bakterien, mit:
einer porösen Oxidkeramik (101); und
einem Laser (103) zum Aussenden von Laserlichtpulsen auf die Oxidkeramik (101) mit einer Energie pro Fläche zwischen 1,8×10⁻¹⁰ J/cm² und 1,5 J/cm².

2. Lasersystem (100) nach Anspruch 1, wobei der Laser (103) ausgebildet ist, die Laserlichtpulse jeweils mit einer Dauer von unter 400 fs auszusenden.

3. Lasersystem (100) nach einem der vorangehenden Ansprüche, wobei der Laser (103) ausgebildet ist, die Laserlichtpulse mit einer Wiederholrate von mehr als 10 MHz oder 80 MHz auszusenden.

4. Lasersystem (100) nach einem der vorangehenden Ansprüche, wobei der Laser (103) ausgebildet ist, die Laserlichtpulse jeweils mit einer Flächenleistungsdichte von über 1 kW/cm² auf die Oxidkeramik (101) auszusenden.

5. Lasersystem (100) nach einem der vorangehenden Ansprüche, wobei der Laser (103) ein Ti:Sa-Laser ist.

6. Lasersystem (100) nach einem der vorangehenden Ansprüche, wobei die Oxidkeramik (101) offene Poren (119) mit einer Porengröße zwischen 0,5 pm bis 20 pm aufweist.

7. Lasersystem (100) nach einem der vorangehenden Ansprüche, wobei die Oxidkeramik (101) Kanäle (105) aufweist, die sich von einer Oberfläche (107) in das Innere (109) der Oxidkeramik (101) erstrecken.

8. Lasersystem (100) nach einem der vorangehenden Ansprüche, wobei das Lasersystem (100) eine Fluoreszenzanregungsvorrichtung (113) zum Erzeugen von sichtbarem Fluoreszenzlicht durch die Bakterien auf der Oxidkeramik (101) umfasst.

9. Lasersystem (100) nach Anspruch 8, wobei das Lasersystem (100) eine elektronische Kamera (115) zum Erfassen des Fluoreszenzlichts umfasst.

10. Lasersystem (100) nach Anspruch 9, wobei die elektronische Kamera (115) ausgebildet ist, diejenigen Bereiche (117) der Oxidkeramik (101) zu erfassen, von denen das Fluoreszenzlicht ausgesendet wird.

11. Lasersystem (100) nach Anspruch 10, wobei das Lasersystem (100) eine Richtvorrichtung (123) zum automatischen Richten der Laserpulse auf die erfassten Bereiche (117) oder eine Aktivierungsvorrichtung (125) zum Aktivieren des Lasers (103) umfasst, wenn eine Fluoreszenz erfasst wird.

12. Lasersystem (100) nach einem der Ansprüche 9 bis 11, wobei das Lasersystem (100) ausgebildet ist, die Energie pro Fläche der Laserpulse auf Basis einer Intensität des erfassten Fluoreszenzlichtes zu steuern.

13. Lasersystem (100) nach einem der vorangehenden Ansprüche, wobei das Lasersystem (100) einen Polarisator (111) zum Polarisieren des Laserlichts umfasst.

14. Lasersystem (100) nach Anspruch 13, wobei der Polarisator (111) drehbar ist.

15. Laserverfahren (100) zum Entfernen von Bakterien von einer porösen Oxidkeramik (101), mit dem Schritt:
- Aussenden (S101) von Laserlichtpulsen auf die Oxidkeramik (101) mit einer Energie pro Fläche zwischen 1,8×10⁻¹⁰ J/cm² und 1,5 J/cm².
